# FASCICULE DE BREVET EUROPEEN

(11) **EP 3 221 000 B1**
(45) Date de publication et mention de la délivrance du brevet: **09.01.2019**
(21) Numéro de dépôt: 15798056.6
(22) Date de dépôt: 19.11.2015
(51) Int. Cl.: H05K 3/06, H05K 3/46, A61N 1/05, A61N 1/02, A61N 1/36, H05K 3/00

(54) **PROCEDE DE FABRICATION D'IMPLANT POUR LA STIMULATION ELECTRIQUE FOCALE D'UNE STRUCTURE NERVEUSE**
VERFAHREN ZUR HERSTELLUNG EINES IMPLANTATS ZUR FOKALEN ELEKTRISCHEN STIMULATION EINER NERVENSTRUKTUR
PROCESS FOR MANUFACTURING AN IMPLANT FOR FOCAL ELECTRICAL STIMULATION OF A NERVOUS STRUCTURE

(30) Priorité: 19.11.2014 FR 1461210
(43) Date de publication de la demande: 27.09.2017
(73) Titulaire: CHAMBRE DE COMMERCE ET D'INDUSTRIE DE REGION PARIS ILE DE FRANCE (ESIEE PARIS), 93160 Noisy le Grand (FR); Institut National de la Santé et de la Recherche Médicale (INSERM), 75013 Paris (FR); Université Pierre et Marie Curie (Paris 6), 75005 Paris (FR); Centre National de la Recherche Scientifique (CNRS), 75016 Paris (FR)
(72) Inventeur: ROUSSEAU, Lionel, 94170 Le Perreux sur Marne (FR); LISSORGUES, Gaëlle, 94170 Le Perreux-sur-Marne (FR); COTTANCE, Myline, 94310 Orly (FR); PICAUD, Serge, 77210 Avon (FR); DEGARDIN, Julie, 91130 Ris-Orangis (FR)
(74) Mandataire: Regimbeau
(86) Numéro de dépôt international: PCT/EP2015/077158
(87) Numéro de publication internationale: WO 2016/079263

(56) Documents cités:
- WO-A1-2011/135273
- WO-A1-2014/126927
- WO-A2-2004/073547
- US-A1- 2014 121 738

## Description

La présente invention est relative aux implants pour la stimulation électrique focale d'une structure nerveuse.

Elle propose en particulier un procédé de fabrication d'un implant de ce type, ainsi qu'une nouvelle structure d'implant.

### DOMAINE TECHNIQUE GÉNÉRAL ET ART ANTÉRIEUR

Les implants permettant la stimulation électrique de structures nerveuses au moyen de réseaux de macro ou microélectrodes connaissent aujourd'hui un fort développement.

Il est par exemple désormais courant d'appareiller avec des implants cochléaires des patients qui sont atteints d'une perte de l'ouïe suite à la détérioration du système marteau et enclume ; également, les premiers implants rétiniens sont en test clinique sur l'homme, pour les personnes ayant perdu la vue, suite à des maladies neurodégénératives comme la DMLA ou la rétinopathie pigmentaire. Des implants corticaux ont également déjà été proposés. Les avancées menées ces dernières années sur les implants de stimulation électrique de structures nerveuses permettent d'envisager de traiter de plus en plus de pathologies et handicaps moteurs ou sensoriels à l'aide de ces techniques.

Une des limitations actuelles des implants de stimulation est la maitrise de la focalisation des signaux de stimulation. Dans le cas des implants rétiniens par exemple, il a été démontré que pour retrouver une vision quasi normale, il était nécessaire de développer des implants de 600 électrodes ou plus. Mais si la focalisation des courants de stimulation n'est pas correctement réalisée, des électrodes contiguës stimulent les mêmes neurones, sans bénéfice pour le patient.

Il a déjà été proposé dans la demande de brevet FR 2.959.420 des structures d'implants rétiniens comportant sur un support
- un réseau de cavités au fond desquelles sont disposées des microélectrodes de stimulation et
- une grille métallique entourant les cavités, au-dessus des microélectrodes.

Une telle structure permet, notamment lorsque la grille est utilisée comme plan de masse, d'obtenir une stimulation focale des cellules de la rétine. Lors d'une stimulation par une électrode, seuls les neurones qui sont dans la cavité de ladite électrode sont activés et aucun neurone dans les autres cavités n'est stimulé.

À ce jour, ces implants tridimensionnels sont généralement fabriqués au moyen d'un moule 3D en silicium à pyramides ou pointes tronquées, sur lequel un polymère est déposé afin de prendre la forme 3D. Les électrodes et la grille de masse sont déposées à certains endroits du moule et l'ensemble est ensuite recouvert par un polymère. Une gravure localisée est réalisée sur ce polymère pour créer les trous pour les électrodes, la grille et les contacts, puis un métal est déposé pour former lesdites électrodes, la grille et les contacts. Une dernière couche de polymère est ensuite déposée sur l'ensemble de la plaquette. Après une dernière gravure, le polymère est décollé du substrat. La libération de l'implant 3D ainsi réalisé se fait par destruction du moule.

Cette technologie de fabrication a toutefois un certain nombre de limitations.

En particulier, elle nécessite la destruction des moules qui ne sont pas réutilisables et sont détruits en fin de procédé pour libérer les implants.

En outre, si cette technologie est adaptée dans le cas d'un faible nombre d'électrodes (quelques dizaines), elle s'avère difficilement compatible avec un nombre d'électrodes plus important.

Également, elle ne permet de réaliser que des implants souples à base de polymère et ne permet pas la réalisation d'implants à substrats rigides amincis en fin de procédé pour devenir souples.

Également encore, cette technologie de fabrication limite fortement les formes qu'il est possible de donner aux cavités de l'implant (ces formes étant soit carrées, soit rondes).

### PRÉSENTATION GÉNÉRALE DE L'INVENTION

Un but général de l'invention est de proposer un procédé de fabrication d'un implant à cavités et réseau métallique ou polymère, isolé ou non, qui ne présente pas les inconvénients des techniques antérieures.

Notamment, un but de l'invention est de proposer un procédé de fabrication qui soit peu onéreux et facile à industrialiser.

Un autre but de l'invention est de proposer un procédé de fabrication particulièrement adapté à la fabrication d'implants comportant un grand nombre d'électrodes.

Un autre but également de l'invention est de proposer un procédé de fabrication qui soit adapté à la réalisation d'implants souples ou rigides.

Un autre but encore est de permettre de nombreuses géométries de cavités pour un implant.

Selon un premier aspect, l'invention propose quant à elle un procédé de fabrication d'au moins un implant pour la stimulation électrique focale d'une structure nerveuse, ledit implant comportant sur un support un réseau formant des cavités au fond desquelles sont disposées des microélectrodes, les cavités étant délimitées par des parois dressées et situées autour des microélectrodes.

De façon remarquable, le support est préalablement réalisé en mettant en oeuvre des étapes dans l'ordre suivant:
- dépôt et gravure, de façon planaire, dans un même plan de base, sur un substrat, des contacts électriques, des pistes électriques et des microélectrodes, des premières pistes électriques reliant les microélectrodes et les contacts électriques, des deuxièmes pistes électriques étant reliées à une masse,
- dépôt d'une couche de polymère sur le substrat, les contacts électriques, les pistes électriques et les microélectrodes,
- gravure de la couche de polymère afin de réaliser des ouvertures au niveau des contacts électriques et des microélectrodes,
- réalisation des parois dressées formant les cavités autour des microélectrodes.

Les électrodes peuvent être des électrodes de stimulation ou de réception.

Le substrat peut être un substrat rigide en un matériau amorphe, de type silicium verre ou analogue.

Les implants 3D sont ensuite décollés du substrat dans le cas de l'implant souple.

Ces technologies peuvent également être réalisées directement sur un substrat rigide qui sera aminci et découpé pour donner les caractéristiques pour être implanté ou utilisé, suivant l'amincissement, l'implant sera plus ou moins rigide.

Avantageusement, on réalise des parois formant les cavités perpendiculairement à la surface de l'implant. Par « perpendiculairement » ou « perpendiculaire », on entend ici et dans tout le présent texte des parois pouvant former des angles par exemple de 85° à 95° par rapport à la surface de l'implant.

Dans une première réalisation d'un premier mode de réalisation, le substrat comporte une première couche de polymère, et le dépôt et la gravure, de façon planaire, dans un même plan de base, de contacts électriques, de pistes électriques et de microélectrodes, sont réalisés sur la première couche de polymère.

Dans ce cas, on grave les deux niveaux de polymère pour définir la forme de l'implant avant de réaliser les parois dressées.

Dans une deuxième réalisation d'un premier mode de réalisation, le support est préalablement réalisé en déposant sur le substrat une électrode, suivi du dépôt et de la gravure des contacts électriques et des pistes électriques, et du dépôt d'une couche de polymère après élimination du substrat qui suit la gravure chimique.

Dans le premier mode de réalisation, on dépose une couche d'accroche métallique située au moins en partie sur la couche de polymère, une électrodéposition est réalisée afin de former des parois métalliques entourant les cavités, lesdites parois électrodéposées constituant au moins en partie un réseau métallique entourant les cavités.

Il est utilisé ici par exemple le même métal entre les parois métalliques et la couche d'accroche métallique.

Avantageusement, l'électrodéposition est précédée du dépôt d'un moule rapporté sur ledit support, ledit moule étant ensuite supprimé.

Avantageusement, on enlève le moule puis la couche d'accroche métallique par gravure chimique.

Avantageusement dans une variante du premier mode de réalisation, on électrodépose sur ledit support une couche métallique, on dissout un masque qui a servi à guider la croissance du métal puis on dissout la couche d'accroche métallique pour réaliser ledit réseau métallique.

Avantageusement, le moule est un moule de résine.

L'utilisation d'un tel moule de résine a de nombreux avantages.

Elle permet de réduire la quantité de métal utilisé.

Elle permet en outre de réaliser des formes à géométrie contrôlée et notamment d'obtenir une résolution submicronique.

Dans un second mode de réalisation, on dépose des parois en polymère afin de former au moins en partie un réseau en polymère entourant les cavités.

Dans une réalisation, les parois formant le réseau sont reliées aux pistes électriques reliées à la masse.

Dans une autre réalisation, les parois formant le réseau ne sont pas reliées aux pistes électriques reliées à la masse. En d'autres termes, dans ce cas, les parois sont isolantes et peuvent être dans ce cas en métal ou en polymère.

Avantageusement, on recouvre d'une couche isolante (polymère) les parois et les microélectrodes afin de les isoler, cette couche de polymère étant ensuite ouverte, par exemple par gravure laser, sur une partie supérieure des microélectrodes et sur une partie supérieure des parois métalliques.

On utilise ici un procédé de type dépôt conforme comme par exemple le parylène.

Dans ce cas, la masse reliée aux parois métalliques est à une autre hauteur que celle de la microélectrode sur le support.

On peut déposer par exemple à titre illustratif et non limitatif une densité de microélectrodes/mm² supérieure à une densité minimale de 100 électrodes/mm².

La présente invention concerne également un implant susceptible d'être obtenu par le procédé défini ci-avant.

La présente invention concerne également un implant pour la stimulation ou réception électrique focale d'une structure nerveuse, ledit implant comportant sur un support un réseau de cavités au fond desquelles sont disposées des microélectrodes, les cavités étant délimitées par des parois dressées et situées autour des microélectrodes.

De façon remarquable, le support comporte de façon planaire, dans un même plan de base, sur une première couche de polymère, des contacts électriques, des pistes électriques et les microélectrodes, des premières pistes électriques reliant les microélectrodes et les contacts électriques, des deuxièmes pistes électriques reliées à une masse.

En outre, le support comporte une deuxième couche de polymère recouvrant les contacts électriques, les pistes électriques et les microélectrodes, la deuxième couche de polymère étant gravée et présentant des ouvertures au niveau des contacts électriques et des microélectrodes, des parois formant les cavités en saillie ou au-dessus de la couche de polymère.

Avantageusement, les parois formant les cavités sont perpendiculaires à la deuxième couche de polymère.

Les parois formant les cavités pourraient aussi être de forme intérieure évasée ou en pente.

Dans un premier mode de réalisation, les parois formant les cavités sont métalliques.

Dans un second mode de réalisation, les parois formant les cavités sont en polymère.

Avantageusement, l'implant comporte une pluralité d'éléments électriques, tels que des photodiodes ou des transistors FETs, intégrés dans le support et reliés par des pistes électriques d'une part à une microélectrode, et d'autre part à la structure tridimensionnelle métallique ou en polymère qui constitue le réseau. Cela est en partie dû à l'utilisation d'un procédé planaire dans un même plan de base P.

Les photodiodes permettent de convertir la lumière en électricité qu'elles transmettent à l'électrode.

Par exemple, les photodiodes peuvent être des photodiodes Zener.

Avantageusement, les parois formant les cavités sont de forme générale en nid d'abeille.

Avantageusement, les parois formant les cavités sont de forme hexagonale.

Avantageusement, les parois formant les cavités sont circulaires et/ou triangulaires et/ou rectangulaires.

Dans une variante de réalisation, les parois des cavités sont séparées et électriquement indépendantes les unes des autres.

Par exemple, les parois formant les cavités ont des hauteurs de 10 µm et inférieure à 100 µm, et préférentiellement entre 30 et 40 µm.

Par exemple, les parois formant les cavités ont des épaisseurs de 2 à 50 µm et préférentiellement entre 8 et 10 µm. Par exemple, la densité des électrodes est associée à un pas entre chaque électrode qui peut être de 70 µm pour une répartition hexagonale.

### PRÉSENTATION DES FIGURES

D'autres caractéristiques et avantages de l'invention ressortiront encore de la description qui suit, laquelle est purement illustrative et non limitative, et doit être lue en regard des figures annexées sur lesquelles :
- La figure 1 est une représentation en perspective d'un implant à cavité qui peut être fabriqué au moyen d'une mise en oeuvre possible du procédé proposé ;
- La figure 2 est une représentation en vue de dessus de la structure générale d'un tel implant ;
- Les figures 3a à 3h illustrent un mode de mise en oeuvre possible pour le procédé proposé par l'invention ;
- Les figures 4a à 4i illustrent un autre mode de mise en oeuvre possible pour le procédé proposé par l'invention ;
- Les figures 5a à 5j illustrent un autre mode de mise en oeuvre encore possible pour le procédé proposé par l'invention ;
- Les figures 6a et 6b illustrent des exemples de formes de cavités qui peuvent être obtenues au moyen d'un procédé tel qu'illustré sur les figures 3a à 3h, 4a à 4i et 5a à 5j ;
- La figure 7 est une vue de dessus illustrant une structure d'implant à photodiode sur laquelle une électrodéposition est susceptible d'être réalisée au moyen d'un procédé tel qu'illustré sur les figures 3a à 3h, 4a à 4i et 5a à 5j ;
- Les figures 8a et 8b illustrent une vue en coupe et une vue de dessus d'un implant conforme à un autre mode de réalisation possible pour l'invention.

### EXEMPLES DE MODES DE MISES EN ŒUVRE ET DE RÉALISATION

### Structure générale d'un implant

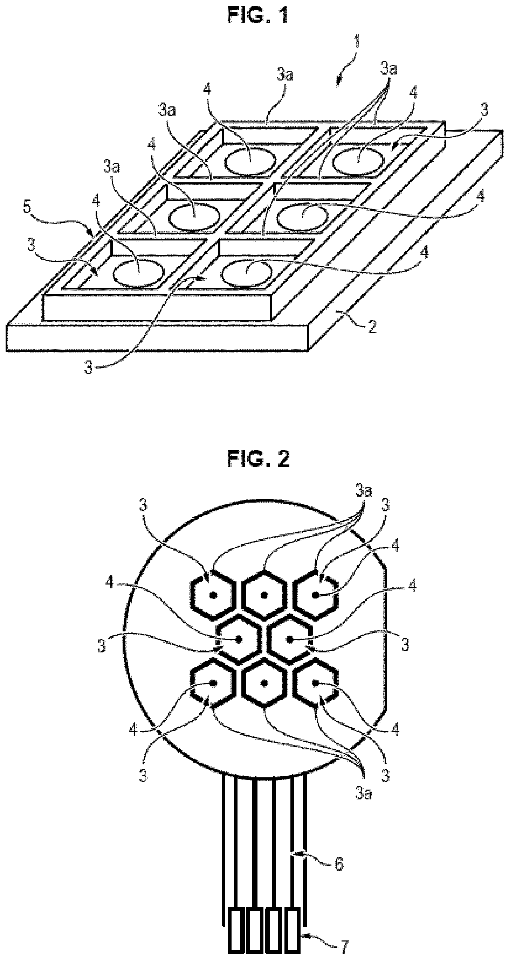

L'implant 1 qui est illustré sur les figures 1 et 2 comporte un substrat 2 électriquement isolant sur lequel est formé un réseau 5 de cavités 3. Des électrodes de stimulation 4 sont disposées au fond de ces cavités 3.

Les parois 3a qui définissent le réseau 5 et les cavités 3 sont métalliques et/ou en matériau polymère. Elles s'étendent en saillie par rapport au plan des électrodes 4, le cas échéant en étant au moins en partie au-dessus de celles-ci.

Le terme 'réseau' utilisé dans toute la description représente un ensemble de parois entrelacées.

Le réseau peut s'apparenter dans certains modes de réalisation à une grille avec des parois en polymère ou encore à une grille rigide avec des parois métalliques. En d'autres termes, les parois présentent une certaine épaisseur et sont ici minces. L'épaisseur des parois est plus faible que la largeur des cavités (ou le diamètre des cavités pour des cavités rondes).

Ce réseau peut être une grille de masse (c'est-à-dire un réseau relié à la masse), ou pas.

Le terme paroi utilisé par la suite désigne un muret ou pilier qui entoure l'électrode 4 et comporte des faces perpendiculaires ou dressées par rapport au fond des cavités (une intérieure et une extérieure) et un sommet. En fonction des réalisations, sachant que la face intérieure entoure l'électrode 4, la face extérieure peut ou non également constituer la face intérieure d'une autre cavité et entourer une autre électrode (cf. figures 1 et 2).

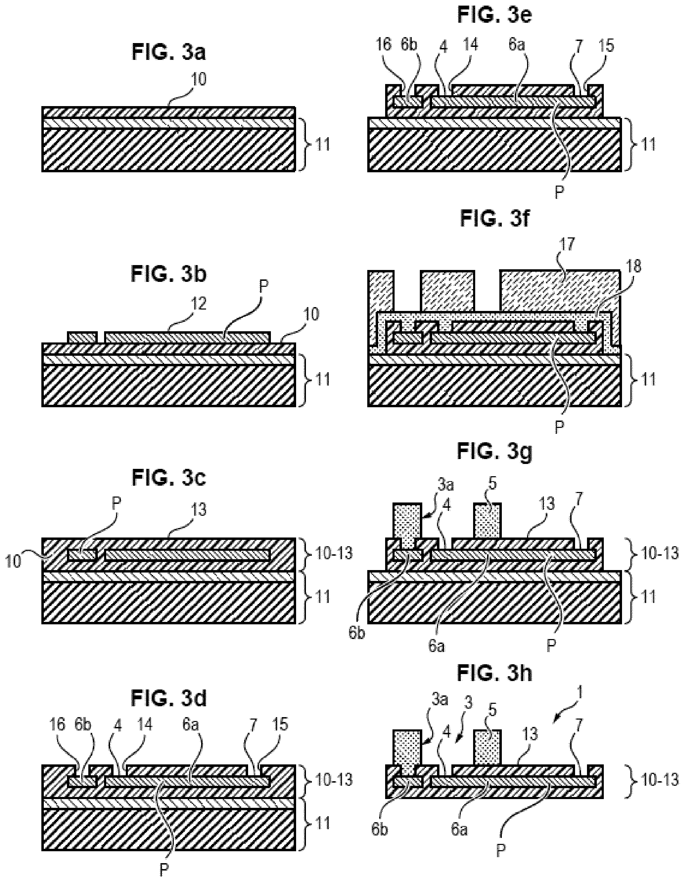

Ces faces sont parallèles et identiques dans les modes de réalisation des figures 3a à 4e, et 8a et 8b, tandis que le sommet leur est perpendiculaire. D'autres modes de réalisation sont bien entendu envisageables. Par exemple les faces intérieures et extérieures peuvent présenter des inclinaisons différentes.

Les électrodes 4 sont reliées par des pistes électriques 6, 6a, 6b à des contacts électriques 7 pour la commande des stimulations électriques par lesdites électrodes.

Des premières pistes 6a relient électriquement les microélectrodes 4 et des contacts électriques 7.

Des deuxièmes pistes électriques 6b séparées des premières pistes 6a et déposées ici en même temps que les premières pistes 6a sur la couche 10 ou sur la couche 11, sont reliées électriquement à une masse et sont à distance des microélectrodes 4.

Les pistes 6, 6a, 6b, les microélectrodes 4 et les contacts électriques 7 sont dans un même plan du support, ce qui permet un meilleur rendement pour la fabrication et une plus grande souplesse pour l'implant.

À titre purement illustratif et non limitatif, il est donné ci-après des dimensions géométriques des éléments caractéristiques du support.

Les électrodes sont typiquement d'une épaisseur inférieure à 10 µm, et d'une dimension dans le plan du substrat allant de quelques micromètres à une centaine de micromètres.

Le réseau 5 a quant à lui typiquement une hauteur supérieure à 10 µm et inférieure à 100 µm. Elle est préférentiellement entre 30 et 40 µm.

La largeur ou épaisseur des parois 3a que ledit réseau 5 définit va par exemple de 2 µm à une cinquante de micromètres. De façon préférentielle, cette largeur est comprise entre 8 et 10 µm.

Les pistes électriques 6a, 6b ont par exemple une largeur de l'ordre de 10 µm et une épaisseur (ou hauteur) de 1 à 5 µm.

Le substrat 2 a une épaisseur (ou hauteur suivant la verticale quand le substrat 2 est horizontal) de l'ordre de quelques dizaines de micromètres.

### Exemple de procédé de fabrication d'un implant à électrodes métalliques

Une structure d'implant du type de celle illustrée sur les figures 1 et 2 est par exemple réalisée de la façon illustrée sur les figures 3a à 3h.

Dans une première étape (figure 3a), une première couche isolante électriquement, ici par exemple une couche de polymère 10, est déposée sur un substrat plan 11 et fait partie, par souci de simplification, de ce substrat. Il n'est pas utilisé de moule à ce stade là pour conférer une forme particulière tridimensionnelle à l'implant final.

La couche 10 est par exemple une couche de 10 à 30 µm d'épaisseur d'un polymère.

Ce polymère peut être un polymère biocompatible de type polyimide ou d'un parylène.

Cette première couche pourrait être également en matériau non polymère tel qu'en oxyde de silicium SiO2.

Le substrat plan 11 est en un matériau rigide amorphe. Il est par exemple constitué par un substrat de Si/SiO2 (cas de la représentation sur les figures 3a à 3h), un substrat de verre ou un substrat de métal.

Dans une deuxième étape (figure 3b), on réalise par dépôt d'une couche métallisée 12, puis gravure, les contacts électriques 7, pistes électriques 6a, 6b et microélectrodes 4 de façon planaire sur la couche de polymère 10 (c'est-à-dire qu'ils appartiennent au même plan de base P horizontal ici).

Le plan de base P comporte une épaisseur dans laquelle se trouve les contacts électriques 7, les pistes électriques 6a, 6b et les microélectrodes 4, et est superposé au substrat isolant plan.

Les métaux utilisés sont par exemple des métaux qui sont biocompatibles (or, platine) et qui s'électrodéposent.

Il est également possible de modifier les électrodes afin de faire baisser les impédances, en utilisant par exemple du black Pt (« platine noir »),

Un métal non biocompatible pourrait le cas échéant également être utilisé, mais nécessiterait d'être recouvert d'un métal bio compatible et de montrer qu'il n'y aurait pas de contact à terme avec celui-ci.

Une seconde couche isolante électriquement 13 est ensuite déposée sur l'ensemble de la plaquette ainsi réalisée (figure 3c).

Cette couche 13 est composée, par exemple, du même polymère que celui de la couche 10. Son épaisseur est du même ordre de grandeur que l'épaisseur de la couche 10.

Cette seconde couche pourrait être également en matériau non polymère tel qu'en oxyde de silicium SiO₂.

La métallisation des pistes électriques 6a, 6b, contacts électriques 7, et électrodes 4 se trouve alors noyée dans le polymère des couches 10 et 13, qui forment alors ensemble une structure plane.

Une première gravure dans le polymère permet d'ouvrir les électrodes 4 (ouvertures 14), les contacts électriques 7 (ouvertures 15), ainsi que les pistes électriques 6a, 6b (ouvertures 16).

Les ouvertures 16 permettent de relier les parois métalliques 3a aux pistes 6b (figure 3d).

Une seconde gravure peut également être prévue pour donner à l'implant sa forme générale externe (figure 3e). Cette étape de gravure externe peut néanmoins être prévue ultérieurement.

Dans le cas où l'on forme un réseau métallique, un moule en résine 17 est ensuite déposé sur la plaquette obtenue à l'issue de l'étape 3d et 3e (figure 3f).

Ce moule 17 est destiné à guider la croissance électrolytique du métal qui va constituer le réseau de masse et définir la cavité autour de l'électrode. Il définit en creux les parois 3a dressées des cavités 3, lesquelles vont également constituer le réseau métallique 5.

On notera que pour permettre la croissance électrolytique, une couche d'accroche 18 est déposée préalablement au dépôt du moule 17.

La couche d'accroche est avantageusement du même matériau que celui du métal du réseau.

La résine du moule 17 est, en fonction de la polarité du masque, soit une résine positive, soit une résine négative. Cette résine doit pouvoir être dissoute en fin de procédé sans dégrader les deux couches de polymère formant l'implant. La résine utilisée est par exemple la résine commercialisée par la société Clariant sous la dénomination AZ125 NxT. Son épaisseur (hauteur ou dimension verticale) est typiquement de l'ordre du double de l'épaisseur de métal de la couche 12 déposée (typiquement 50 µm).

Après croissance électrolytique du métal du réseau 5 dans ledit moule, le moule en résine est dissout (figure 3g) (gravure chimique). La couche d'accroche 18 du même métal que celui des parois 3a est éliminée par gravure légère après que le moule 17 ait été détruit.

L'implant réalisé est ensuite décollé par rapport au substrat plan 11.

On obtient alors l'implant 3D tel que représenté sur la figure 3h. Cet implant est un implant souple ayant une structure plane noyée dans le polymère des couches 10 et 13 qui constituent alors ensemble le substrat 2 de l'implant 1, et des parois dressées 3a au-dessus (ou en saillie) de la structure plane.

### Exemple de procédé de fabrication d'un implant à électrodes en carbone (par exemple en diamant ou en graphène)

En variante, d'autres matériaux que des métaux peuvent être utilisés pour les électrodes 4.

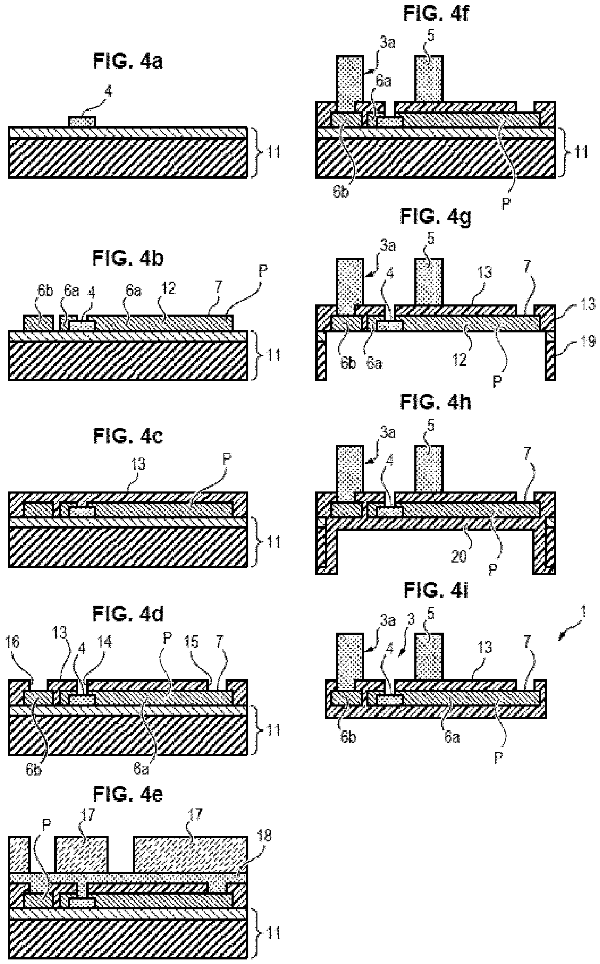

Notamment, pour des électrodes 4 en carbone (par exemple en diamant ou en graphène) qui nécessitent un dépôt à haute température, les étapes de fabrication peuvent être les suivantes :
- dépôt des électrodes 4 sur un substrat plan 11 adapté (substrat Si/SiO₂ par exemple) (figure 4a) ;
- dépôt de façon planaire, dans un même plan de base P, sur le substrat 11 d'une couche de métallisation 12 et gravure de celle-ci pour définir les pistes électriques 6a, 6b (en particulier celles connectant les électrodes 4) et les contacts électriques 7 (figure 4b), l'électrode 4 pouvant être plus petite en hauteur que le plan des pistes électriques et des contacts électriques 7 ;
- dépôt d'une couche de polymère 13 sur l'ensemble de la plaquette (figure 4c) ;
- gravure pour ouvrir les électrodes 4, les pistes électriques 6a, 6b et les contacts électriques 7 (ouvertures 14, 15 et 16) (figure 4d) ;
- après dépôt d'une couche d'accroche 18, dépôt d'un moule de résine 17 (figure 4e) ;
- électrodéposition du métal du réseau dans les ouvertures dudit moule et dissolution de la résine puis gravure de la couche d'accroche 18 pour la supprimer, le métal ainsi libéré constituant les parois 3a dressées des cavités 3 et le réseau de référence 5 (figure 4f) ;
- gravure de la face arrière du substrat 11 sur toute son épaisseur afin d'accéder aux couches déposées sur la face avant tout en laissant un maillage de rigidification 19 (figure 4g) ;
- dépôt d'un polymère 20 tel qu'un parylène sur la face arrière de l'implant afin d'isoler celle-ci (figure 4h) correspondant à la face arrière du plan de base P;
- découpe laser de l'ensemble afin de donner à l'implant la forme générale souhaitée (figure 4i).

On obtient alors l'implant 3D tel que représenté sur la figure 4i. Cet implant est un implant souple ayant une structure plane noyée dans le polymère des couches 20 et 13 qui constituent alors ensemble le substrat 2 de l'implant 1, et des parois dressées 3a au-dessus (ou en saillie) de la structure plane.

On notera que le procédé pourrait ne pas comprendre les étapes 4g et 4h de façon à permettre de disposer d'un implant rigide au lieu d'un implant souple.

L'implant obtenu est alors un implant rigide dont le substrat 2 est constitué par la couche 11.

L'exemple de mise en oeuvre qui vient d'être décrit concerne le cas d'implant en diamant ou en graphène. Comme on l'aura compris néanmoins, les étapes 4c à 4f, voire 4g et 4i, peuvent également être mises en oeuvre dans le cas d'électrodes métalliques déposées et gravées avec les pistes électriques et contacts électriques préalablement à la mise en oeuvre de l'étape 4c afin de réaliser des implants 3D.

### Autre mise en oeuvre possible

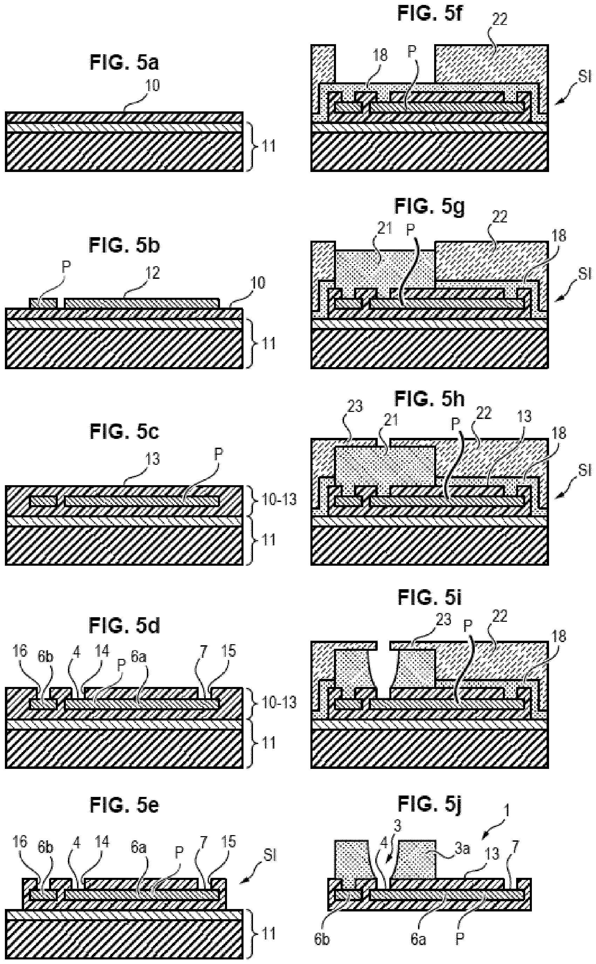

Une autre variante de mise en oeuvre est illustrée sur les figures 5a à 5j.

Les étapes illustrées sur les figures 5a à 5e sont identiques à celles déjà présentées aux figures 3a à 3e.

On dispose ainsi à l'issue de l'étape 5e d'un support intégrant la métallisation des électrodes 4, pistes électriques 6a, 6b et contacts électriques 7 (support intégré SI) de façon planaire sur le substrat 11 dans un même plan de base P.

En variante encore, le support intégré SI peut être réalisé indépendamment chez un fondeur externe.

Une fois ce support obtenu, on dépose sur celui-ci, au niveau de la zone de l'implant 1 destinée à être couverte par le réseau 5, une couche de métallisation 21 d'une épaisseur correspondant à celle des futures cavités 3 (étape 5g).

Le dépôt se fait par électrodéposition.

Une couche d'accroche métallique 18 et un moule 22 auront préalablement été mis en place sur le support intégré.

La couche d'accroche métallique 18 peut recouvrir l'ensemble de la surface avant du support intégré SI.

Elle peut être uniquement localisée au niveau de la ou des zones du support SI sur lesquelles l'électrodéposition est mise en oeuvre.

Une fois l'électrodéposition terminée, on réalise un masquage complémentaire 23 de la couche métallique 21 ainsi déposée (figure 5h), puis une gravure de ladite couche par photolithographie (figure 5i).

On notera ici que la photolithographie ainsi réalisée permet en particulier des formes intérieures de cavité évasées ou en pente.

Dans une dernière étape, les moules 22 et les masques 23 et le cas échéant la couche support 11, sont supprimés chimiquement ou mécaniquement pour obtenir l'implant 1 (figure 5j).

### Géométries 3D des implants et autres variantes

Comme on l'aura compris, les modes de mise en oeuvre qui viennent d'être décrits permettent de nombreuses formes pour le réseau de référence 5, puisqu'il est possible de donner de nombreuses géométries 3D aux formes en creux des différents moules 17, 22 et du masque 23.

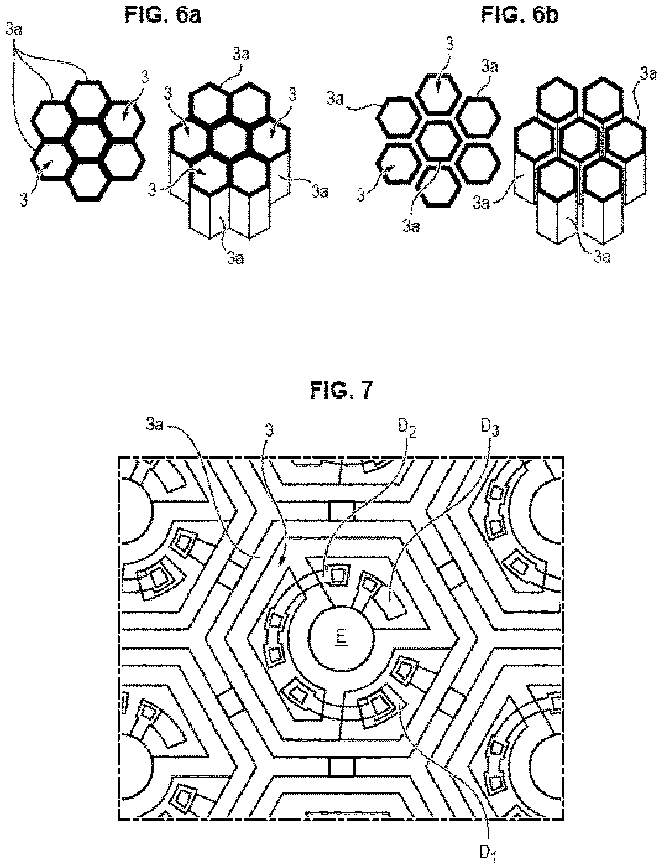

Par exemple, comme l'illustrent les figures 6a et 6b, les cavités 3 peuvent être des structures en nids d'abeilles.

De nombreuses autres formes sont bien entendu possibles pour les cavités : hexagonales, rondes, triangulaires, etc..., le cas échéant avec des formes intérieures 3D particulières selon les formes intérieures définies par les moules et/ou les techniques de réalisation utilisées.

Par ailleurs, la technologie proposée permet de réaliser les réseaux cavités soit avec des cavités 3 accolées les unes aux autres (avec alors le même potentiel de référence pour chacune) comme illustré figure 6a, soit au contraire avec des cavités 3 séparées comme illustré figure 6b (ce qui permet le cas échéant de jouer sur les potentiels de référence qu'on leur donne).

En variante encore, ainsi que l'illustre la figure 7, la structure sur laquelle l'électrodéposition est réalisée lors des étapes 3f, 4e, 5f, et suivantes, peut être une structure à semi-conducteurs comportant une pluralité de photodiodes ou transistors FETs D1, D2, D3 reliées d'un côté à l'électrode E de la cavité 3 et de l'autre au métal qui entoure l'électrode E et qui définit ladite cavité 3.

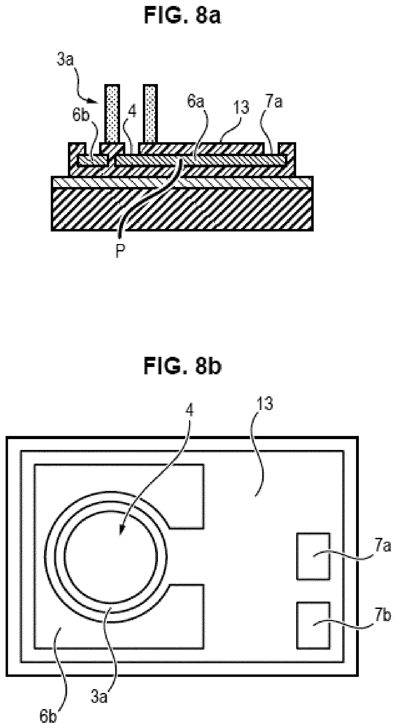

Les figures 8a et 8b représentent un autre mode de réalisation possible dans lequel les parois 3a sont situées à distance des premières pistes 6a et des deuxièmes pistes 6b et dans lequel le réseau 5 ne joue pas le rôle de masse.

Ces parois 3a peuvent être en matériau polymère, ce qui constitue le deuxième mode de réalisation par opposition au premier mode de réalisation dans lequel les parois sont métalliques.

Cependant, les figures 8a et 8b illustrent également le cas d'une variante du premier mode de réalisation dans laquelle les parois dressées 3a sont en matériau métallique (et réalisées par électrodéposition).

Dans le cas où les parois 3a sont en matériau polymère, on réalise ces parois 3a par déposition d'une résine épaisse et biocompatible de type SU8 (MicroChem). Cette résine de type époxy permet de réaliser des structures de plusieurs dizaines de microns de hauteur. Une exposition aux UV permet de venir réticuler les zones qui sont exposées et lors du développement de dissoudre les zones non-réticulées et venir révéler la structure.

Sur la figure 8a, la masse n'est pas en contact avec la paroi 3a située autour de la microélectrode 4. Dans ce cas, les parois 3a peuvent être en métal ou en polymère et jouent le rôle de parois isolantes.

En d'autres termes ici, le réseau n'est pas lié à la masse et n'est pas une grille de masse.

Dans cette configuration d'électrodes le potentiel d'électrode obtenu (quantité de charges en un point) est très important (par exemple de 90 mV, 125 mV et 155 mV à des hauteurs de paroi 3a de : 10 µm, 20 µm, 30 µm).

Il a été constaté par ailleurs que plus la largeur du plan de masse (dimension suivant l'horizontal) est grande, plus le potentiel au niveau de l'électrode est faible donc plus le potentiel d'électrode est mieux réparti dans l'ensemble de la cavité, ce qui permet une meilleure stimulation de la cellule, ce qui s'explique par un retour de courant plus grand avec une plus grande masse.

Il a été constaté par ailleurs que plus le plan de masse se rapproche des électrodes et plus le potentiel maximal au niveau de l'électrode diminue et donc plus le potentiel d'électrode est mieux réparti dans l'ensemble de la cavité, ce qui permet une meilleure stimulation de la cellule. La proximité du plan de masse avec l'électrode assurerait un retour du courant rapide et donc l'accumulation de charges dans le milieu.

Il peut donc être intéressant d'avoir un plan de masse assez large (dont le dimensionnement doit toutefois tenir compte de la résolution spatiale des implants ; par exemple d'une dizaine à une quarantaine de micromètres) et proche de l'électrode (par exemple une dizaine de micromètres) pour permettre un confinement du courant.

La structure en forme de cavité due à la hauteur des parois (avec une hauteur par exemple de 30 micromètres) permet par ailleurs de générer un potentiel d'électrode plus homogène au niveau de l'électrode 4.

Au contraire, dans les réalisations illustrées sur les figures 3h, 4i, 5j, la masse 6b est en contact avec la paroi 3a située autour de la microélectrode 4. La paroi 3a est, sur ces dessins, métallique.

En d'autres termes ici le réseau est lié à la masse et est un réseau de masse.

Dans ce cas de façon avantageuse, la masse peut être localisée sur une surface supérieure de la paroi dressée 3a située autour de la microélectrode 4 et située au-dessus par rapport au plan du support de la microélectrode 4, par exemple par un polymère recouvrant les faces verticales ou dressées des parois 3a juste au-dessus de la microélectrode 4.

La figure 8b permet d'illustrer le positionnement de la masse qui entoure la microélectrode 4, et permet le retour de courant. Dans ce cas on se retrouve dans une configuration où l'on a un réseau de cavités dans lesquelles des parties de tissu viennent se placer, chaque partie du tissu étant isolée dans la cavité des autres parties de tissu dans les autres cavités, comme viendrait le faire un réseau de micropipette.

Comme déjà évoqué ci-avant, la stimulation d'électrodes avec un plan de masse qui entoure l'électrode 4 et avec une structure tridimensionnelle que constituent les parois permet d'obtenir une stimulation plus focale. L'importance du confinement du courant et le potentiel au niveau de l'électrode 4 dépend de la taille du plan de masse, de sa proximité à l'électrode 4 et de son positionnement par rapport à l'électrode 4. Une stimulation doit être suffisante (c'est-à-dire un potentiel d'électrode élevé au niveau des électrodes) mais aussi localisée (au moyen d'un plan de masse) pour stimuler efficacement et spécifiquement une zone du tissu.

Une structure planaire dans un même plan de base P pour la masse qui entoure l'électrode et avec des parois dressées autour de l'électrode permet d'atteindre cette stimulation avec un potentiel élevé au niveau de l'électrode 4.

Comme illustré ici, il y a des contacts électriques 7a reliés via les premières pistes 6a à l'électrode 4, et il y a des contacts électriques 7b reliés aux deuxièmes pistes 6b (qui ne sont pas en contact avec le réseau isolant).

Sur l'ensemble des figures 1 à 4e et des figures 8a et 8b, les parois dressées 3a sont droites c'est-à-dire perpendiculaires au support ou à la couche de polymère 13. On entend par perpendiculaire comme déjà décrit précédemment des angles allant par exemple de 85° à 95° par rapport à la verticale quand le support est posé à l'horizontale.

Cela permet de présenter plus d'électrodes par unité de surface.

Ainsi grâce à l'invention un meilleur positionnement de l'implant est obtenu lorsque le tissu se recolle (implant sous rétinien).

Par ailleurs, cet implant permet une isolation d'un groupe d'intérêt de cellules nerveuses dans chaque cavité obtenue.

En fonction des propriétés électriques de la paroi (si la paroi est isolante ou conductrice), il est possible de faire varier les niveaux de stimulation électrique de ce groupe d'intérêt.

Ce nouveau procédé de fabrication offre plusieurs avantages.

Il est compatible avec la réalisation d'implants 3D contenant un très grand nombre d'électrodes.

Il permet le maintien d'une structure planaire dans un même plan de base P tout au long de la fabrication de l'implant. Ceci permet d'accroitre les rendements de fabrication.

De nouvelles formes peuvent être envisagées, ainsi par cette technique nous n'avons plus de limitation dans la définition des formes des cavités comme c'était le cas avec les procédés de fabrication de l'état de l'art (carré ou rond). Il est ainsi possible de faire des dispositifs en nid d'abeille, triangulaires, polygones ou autres. Il est également possible de jouer sur les profils des cavités par la modification du profil des moules.

Cette technologie peut s'appliquer à tout type d'implants souples ou rigides.

Le réseau de masse est composé de métal sur toute sa hauteur, ce qui fait que tout le réseau est conducteur dans le cas du premier mode de réalisation. Pour n'avoir que des zones conductrices spécifiques, il est possible de faire un dépôt de polymère puis une ouverture localisée par laser des zones actives.

Il est également possible d'avoir un réseau isolant de parois dressées déposé entre la masse et les microélectrodes comme décrit dans le deuxième mode de réalisation, et dans lequel le réseau ne joue pas le rôle de masse.

La structuration 3D permet de maintenir en place l'implant sans avoir besoin de clous lorsqu'il est placé entre les tissus (exemple : implant sous rétinien).

La structuration 3D particulière associée à ce procédé de fabrication permet de réaliser un réseau formant des cavités au fond desquelles sont situées des photodiodes ou des transistors FETs.

## Revendications

1. Procédé de fabrication d'au moins un implant pour la stimulation ou réception électrique focale d'une structure nerveuse, ledit implant étant du type comportant, sur un support, un réseau (5) formant des cavités (3) au fond desquelles sont disposées des microélectrodes (4), les cavités (3) étant délimitées par des parois dressées (3a) du réseau (5) entourant les électrodes,
**caractérisé en ce que** le support est préalablement réalisé en mettant en oeuvre les étapes suivantes :
- dépôt et gravure, de façon planaire, sur un substrat isolant plan (10, 11) :
des contacts électriques (7), des premières pistes électriques (6a), des deuxièmes pistes électriques (6b) séparées des premières pistes électriques (6a), et des microélectrodes (4) pour que les contacts électriques (7), les premières et les deuxièmes pistes électriques (6a, 6b), et les microélectrodes (4) appartiennent au même plan de base (P),
les premières pistes (6a) reliant électriquement les microélectrodes (4) et les contacts électriques (7),
les deuxièmes pistes électriques (6b) étant reliées électriquement à une masse,
- dépôt d'une couche d'isolant (13) sur le substrat (10, 11) plan, les contacts électriques (7), les pistes électriques (6a, 6b) et les microélectrodes (4),
- gravure de la couche isolante (13) afin de réaliser des ouvertures (14, 15) au niveau des contacts électriques (7) et des microélectrodes (4),
- réalisation d'un réseau de parois (3a) dressées au-dessus de la couche isolante (13) et du plan de base (P), et formant les cavités (3) autour des microélectrodes (4).

2. Procédé selon la revendication 1, **caractérisé en ce que** l'on réalise un réseau de parois (3a) qui forme une grille.

3. Procédé selon l'une des revendications 1 à 2, **caractérisé en ce que** l'on réalise des parois dressées (3a) formant les cavités (3) perpendiculairement à la couche isolante (13).

4. Procédé selon l'une des revendications 1 à 3, **caractérisé en ce que** l'on dépose une couche d'accroche métallique (18) située au moins en partie sur la couche isolante (13), une électrodéposition est réalisée afin de former des parois (3a) métalliques entourant les cavités (3), lesdites parois (3a) électrodéposées constituant au moins en partie un réseau métallique entourant les cavités (3).

5. Procédé selon la revendication 4, **caractérisé en ce que** l'électrodéposition est précédée du dépôt d'un moule rapporté sur ledit support, ledit moule étant ensuite supprimé.

6. Procédé selon l'une des revendications 1 à 5, **caractérisé en ce que** le substrat plan comporte une première couche isolante (10) polymère, et le dépôt et la gravure, de façon planaire, de contacts électriques (7), de pistes électriques (6a, 6b) et de microélectrodes (4), sont réalisés sur la première couche isolante polymère (10).

7. Procédé selon l'une des revendications 1 à 5, **caractérisé en ce que** le support est préalablement réalisé en déposant sur le substrat isolant plan une électrode, puis par dépôt et gravure des contacts électriques (7) et des pistes électriques (6a, 6b), et **en ce qu'**une couche isolante (20) polymère est déposée après élimination du substrat qui suit la gravure chimique.

8. Procédé selon l'une des revendications 4 à 7, **caractérisé en ce que** l'on recouvre d'une couche de polymère les parois (3a) et les microélectrodes afin de les isoler, cette couche de polymère étant ensuite ouverte sur une partie supérieure des microélectrodes et sur une partie supérieure des parois métalliques.

9. Procédé selon l'une des revendications 4 à 8, **caractérisé en ce que** les parois métalliques (3a) sont reliées aux deuxièmes pistes (6b).

10. Procédé selon l'une des revendications 1 à 8, **caractérisé en ce que** l'on dépose des parois (3a) situées à distance des premières pistes (6a) et des deuxièmes pistes (6b).

11. Implant pour la stimulation électrique focale d'une structure nerveuse réalisé selon le procédé défini selon l'une des revendications 1 à 10, ledit implant étant du type comportant, sur un support, un réseau de cavités (3) au fond desquelles sont disposées des microélectrodes (4), les cavités (3) étant délimitées par des parois (3a) dressées, le support comportant de façon planaire sur une première couche isolante (10, 20) plane :
des contacts électriques (7), des premières et des deuxièmes pistes électriques (6a, 6b) et les microélectrodes (4) qui appartiennent tous au même plan de base (P) superposé sur la première couche isolante (10,20) plane,
les premières pistes électriques (6a) reliant les microélectrodes (4) et les contacts électriques (7), les deuxièmes pistes électriques (6b) étant reliées à une masse et étant séparées des premières pistes électriques (6a),
et **caractérisé en ce que** le support comporte :
- une deuxième couche isolante (13) recouvrant les contacts électriques (7), les pistes électriques (6a, 6b) et les microélectrodes (4), la deuxième couche isolante (13) étant gravée et présentant des ouvertures (14, 15) au niveau des contacts électriques (7) et des microélectrodes (4),
- un réseau de parois (3a) dressées au-dessus de la deuxième couche isolante (13) et du plan de base (P), formant les cavités (3) autour des microélectrodes (4).

12. Implant selon la revendication 11, **caractérisé en ce que** le réseau de parois (3a) forme une grille.

13. Implant selon la revendication 11 ou 12, **caractérisé en ce que** les parois (3a) formant les cavités sont perpendiculaires à la deuxième couche isolante (13).

14. Implant selon l'une des revendications 11 à 13, **caractérisé en ce qu'**il comporte une pluralité d'éléments électriques, tels que des photodiodes ou des transistors FETs, intégrés dans le support et reliés par des pistes électriques (6a, 6b) à une microélectrode.

15. Implant selon l'une des revendications 11 à 14, **caractérisé en ce que** les parois (3a) formant les cavités (3) sont métalliques ou en polymère.

## Patentansprüche

1. Verfahren zur Herstellung zumindest eines Implantats zur Stimulation oder zum fokalen elektrischen Empfang einer Nervenstruktur, wobei das Implantat von der Art ist, die ein Netzwerk (5) auf einem Träger umfasst, das Hohlräume (3) bildet, an deren Boden Mikroelektroden (4) angeordnet sind, wobei die Hohlräume (3) durch erhabene Wände (3a) des Netzwerks (5) begrenzt werden, die die Elektroden umgeben,
**dadurch gekennzeichnet, dass** der Träger zuvor mit der Durchführung der folgenden Schritte herstellt wird:
- Aufbringen und planares Ätzen auf einem isolierenden planen Substrat (10, 11):
elektrische Verbindungen (7), erste elektrische Bahnen (6a), zweite elektrische Bahnen (6b), die von den ersten elektrischen Bahnen (6a) getrennt sind, und Mikroelektroden (4), so dass die elektrischen Verbindungen (7), die ersten und zweiten elektrischen Bahnen (6a, 6b) und Mikroelektroden (4) zur gleichen Basisebene (P) gehören,
wobei die ersten Bahnen (6a), die die Mikroelektroden (4) und die elektrischen Verbindungen (7) elektrisch verbinden,
wobei die zweiten elektrischen Bahnen (6b) mit einer Masse elektrisch verbunden sind,
- Aufbringen einer Isolierschicht (13) auf dem planen Substrat (10, 11), der elektrischen Verbindungen (7), der elektrischen Bahnen (6a, 6b) und der Mikroelektroden (4),
- Ätzen der Isolierschicht (13), um Öffnungen (14, 15) in den elektrischen Verbindungen (7) und Mikroelektroden (4) durchzuführen,
- Durchführung eines Netzwerks von Wänden (3a), die über der Isolierschicht (13) und der Basisebene (P) errichtet werden und die Hohlräume (3) um die Mikroelektroden (4) herum bilden.

2. Verfahren nach Anspruch 1, **dadurch gekennzeichnet, dass** ein Netzwerk von Wänden (3a) durchgeführt wird, das ein Gitter bildet.

3. Verfahren nach einem der Ansprüche 1 bis 2, **dadurch gekennzeichnet, dass** erhabene Wände (3a) ausgeführt werden, die die Hohlräume (3) senkrecht zur Isolierschicht (13) bilden.

4. Verfahren nach einem der Ansprüche 1 bis 3, **dadurch gekennzeichnet, dass** eine zumindest teilweise auf der Isolierschicht (13) befindliche Metallhaftschicht (18) aufgebracht wird, eine Elektrobeschichtung durchgeführt wird, um Metallwände (3a) zu bilden, die die Hohlräume (3) umgeben, wobei die elektrobeschichteten Wände (3a) zumindest teilweise ein Metallnetzwerk bilden, das die Hohlräume (3) umgibt.

5. Verfahren nach Anspruch 4, **dadurch gekennzeichnet, dass** vor der Elektrobeschichtung eine Form auf dem Träger aufbringen wird, wobei die Form dann entfernt wird.

6. Verfahren nach einem der Ansprüche 1 bis 5, **dadurch gekennzeichnet, dass** das plane Substrat eine erste Polymer-Isolierschicht (10) umfasst und das Aufbringen und planare Ätzen von elektrischen Verbindungen (7), elektrischen Bahnen (6a, 6b) und Mikroelektroden (4) auf der ersten Polymer-Isolierschicht (10) durchgeführt werden.

7. Verfahren nach einem der Ansprüche 1 bis 5, **dadurch gekennzeichnet, dass** der Träger zuvor durch Aufbringen einer Elektrode auf dem isolierenden planen Substrat, dann Aufbringen und Ätzen der elektrischen Verbindungen (7) und der elektrischen Bahnen (6a, 6b) hergestellt wird, und dass eine Polymer-Isolierschicht (20) nach dem Entfernen des Substrats nach dem chemischen Ätzen aufgebracht wird.

8. Verfahren nach einem der Ansprüche 4 bis 7, **dadurch gekennzeichnet, dass** die Wände (3a) und die Mikroelektroden zur Isolierung mit einer Polymerschicht abgedeckt werden, wobei die Polymerschicht dann auf einem oberen Teil der Mikroelektroden und auf einem oberen Teil der Metallwände geöffnet wird.

9. Verfahren nach einem der Ansprüche 4 bis 8, **dadurch gekennzeichnet, dass** die Metallwände (3a) mit den zweiten Bahnen (6b) verbunden werden.

10. Verfahren nach einem der Ansprüche 1 bis 8, **dadurch gekennzeichnet, dass** Wände (3a) in einem Abstand von den ersten Bahnen (6a) und den zweiten Bahnen (6b) aufgebracht werden.

11. Implantat zur fokalen elektrischen Stimulation einer Nervenstruktur, das nach dem nach einem der Ansprüche 1 bis 10 definierten Verfahren ausgeführt wird, wobei das Implantat von der Art ist, die ein Netzwerk (5) von Hohlräumen (3) auf einem Träger umfasst, an deren Boden Mikroelektroden (4) angeordnet sind, wobei die Hohlräume (3) durch erhabene Wände (3a) begrenzt werden, wobei der Träger auf einer ersten planen Isolierschicht (10, 20) planar Folgendes umfasst:
elektrische Verbindungen (7), erste und zweite elektrische Bahnen (6a, 6b) und die Mikroelektroden (4), die alle zur gleichen Basisebene (P) gehören, die die erste plane Isolierschicht (10, 20) überlagert,
die ersten elektrischen Bahnen (6a), die die Mikroelektroden (4) und die elektrischen Verbindungen (7) verbinden, wobei die zweiten elektrischen Bahnen (6b) mit einer Masse verbunden und von den ersten elektrischen Bahnen (6a) getrennt sind,
und **dadurch gekennzeichnet, dass** der Träger Folgendes umfasst:
- eine zweite Isolierschicht (13), die die elektrischen Verbindungen (7), die elektrischen Bahnen (6a, 6b) und die Mikroelektroden (4) abdeckt, wobei die zweite Isolierschicht (13) geätzt ist und Öffnungen (14, 15) an den elektrischen Verbindungen (7) und den Mikroelektroden (4) aufweist,
- ein Netzwerk von Wänden (3a), die über der zweiten Isolierschicht (13) und der Basisebene (P) erhaben sind und die Hohlräume (3) um die Mikroelektroden (4) herum bilden.

12. Implantat nach Anspruch 11, **dadurch gekennzeichnet, dass** das Netzwerk von Wänden (3a) ein Gitter bildet.

13. Implantat nach Anspruch 11 oder 12, **dadurch gekennzeichnet, dass** die die Hohlräume bildenden Wände (3a) senkrecht zur zweiten Isolierschicht (13) verlaufen.

14. Implantat nach einem der Ansprüche 11 bis 13, **dadurch gekennzeichnet, dass** es eine Mehrzahl von elektrischen Elementen, wie Fotodioden oder FET-Transistoren umfasst, die in den Träger integriert und durch elektrische Bahnen (6a, 6b) mit einer Mikroelektrode verbunden sind.

15. Implantat nach einem der Ansprüche 11 bis 14, **dadurch gekennzeichnet, dass** die die Hohlräume (3) bildenden Wände (3a) aus Metall oder Polymer bestehen.

## Claims

1. A method for manufacturing at least one implant for focal electrical stimulation or reception of a nervous structure, said implant being of the type including, on a support, a network (5) forming cavities (3) at the bottom of which are disposed microelectrodes (4), the cavities (3) being delimited by erected walls (3a) of the network (5) surrounding the electrodes,
**characterized in that** the support is previously prepared by implementing the following steps:
- depositing and etching, in planar fashion, on a planar insulating substrate (10, 11):
the electrical contacts (7), first electrical tracks (6a), second electrical tracks (6b) separated from the first electrical tracks (6a), and microelectrodes (4) so that the electrical contacts (7), first and second electrical tracks (6a, 6b) and microelectrodes (4) belong to the same basic plane (P),
the first electrical tracks (6a) electrically connecting the microelectrodes (4) and electrical contacts (7),
the second electrical tracks (6b) being electrically connected to a ground,
- depositing an insulating layer (13) on the planar substrate (10, 11), electrical contacts (7), electrical tracks (6a, 6b) and microelectrodes (4),
- etching the insulating layer (13) to make openings (14, 15) at the electrical contacts (7) and microelectrodes (4),
- forming a network of erected walls (3a) above the insulating layer (13) and basic plane (P), and forming the cavities (3) around the microelectrodes (4).

2. The method according to claim 1, **characterized in that** a network of walls (3a) which forms a grid is formed.

3. The method according to any of claims 1 to 2, **characterized in that** erected walls (3a) forming the cavities (3) perpendicular to the insulating layer (13) are formed.

4. The method according to any of claims 1 to 3, **characterized in that** a metal bonding layer (18), located at least partly on the insulating layer (13), is deposited, electro-deposition is conducted to form metal walls (3a) surrounding the cavities (3), said electrodeposited walls (3a) forming at least partly a metal network surrounding the cavities (3).

5. The method according to claim 4, **characterized in that** electro-deposition is preceded by the depositing of a mould added onto said support, said mould then being eliminated.

6. The method according to any of claims 1 to 5, **characterized in that** the planar substrate includes a first insulating polymer layer (10), and the depositing and etching, in planar fashion, of electrical contacts (7), electrical tracks (6a, 6b) and microelectrodes (4) are performed on the first insulating polymer layer (10) .

7. The method according to any of claims 1 to 5, **characterized in that** the support is previously prepared by depositing an electrode on the planar insulating substrate, then by depositing and etching electrical contacts (7) and electrical tracks (6a, 6b), and **in that** an insulating polymer layer (20) is deposited after elimination of the substrate which follows chemical etching.

8. The method according to any of claims 4 to 7, **characterized in that** the walls (3a) and microelectrodes are covered with a polymer layer in order to be insulated, this polymer layer then being opened on an upper part of the microelectrodes and on an upper part of the metal walls.

9. The method according to any of claims 4 to 8, **characterized in that** the metal walls (3a) are connected to the second tracks (6b).

10. The method according to any of claims 1 to 8, **characterized in that** walls (3a), located at a distance away from the first tracks (6a) and second tracks (6b), are deposited.

11. An implant for the focal electrical stimulation of a nervous structure, produced according to the method defined according to any of claims 1 to 10, said implant being of the type including, on a support, a network of cavities (3) at the bottom of which are disposed microelectrodes (4), the cavities (3) being delimited by erected walls (3a), the support including, in planar fashion, on a first planar insulating layer (10, 20):
- electrical contacts (7), first and second electrical tracks (6a, 6b) and microelectrodes (4) which all belong to the same basic plane (P) superimposed over the first planar insulating layer (10, 20),
the first electrical tracks (6a) connecting the microelectrodes (4) and the electrical contacts (7), the second electrical tracks (6b) being connected to a ground and being separated from the first electrical tracks (6a),
and **characterized in that** the support includes:
- a second insulating layer (13) covering the electrical contacts (7), electrical tracks (6a, 6b) and microelectrodes (4), the second insulating layer (13) being etched and having openings (14, 15) at the electrical contacts (7) and microelectrodes (4),
- a network of erected walls (3a) above the second insulating layer (13) and basic plane (P), forming the cavities (3) around the microelectrodes (4).

12. The implant according to claim 11, **characterized in that** the network of walls (3a) forms a grid.

13. The implant according to claim 11 or 12, **characterized in that** the walls (3a) forming the cavities are perpendicular to the second insulating layer (13).

14. The implant according to any of claims 11 to 13, **characterized in that** it includes a plurality of electrical elements, such as photodiodes or FET transistors, integrated in the support and connected via electrical tracks (6a, 6b) to a microelectrode.

15. The implant according to any of claims 11 to 14, **characterized in that** the walls (3a) forming the cavities (3) are in metal or polymer.
